# EUROPÄISCHE PATENTSCHRIFT

(11) **EP 4 138 740 B1**
(45) Veröffentlichungstag und Bekanntmachung des Hinweises auf die Patenterteilung: **25.06.2025**
(21) Anmeldenummer: 21720241.5
(22) Anmeldetag: 19.04.2021
(51) Int. Cl.: A61F 2/64, A61F 2/68, A61F 2/70

(54) **VERFAHREN ZUM UEBERTRAGEN VON DATEN**
METHOD FOR TRANSMITTING DATA
PROCÉDÉ DE TRANSMISSION DE DONNÉES

(30) Priorität: 20.04.2020 DE 102020110687
(43) Veröffentlichungstag der Anmeldung: 01.03.2023
(73) Patentinhaber: Otto Bock Healthcare Products GmbH, 1110 Wien (AT)
(72) Erfinder: ANDRUSHCHENKO, Serhii, 2331 Voesendorf (AT); OSSWALD, Fabian, 2511 Pfaffstaetten (AT); HOFMANN, Kerstin, 1070 Wien (AT); AMSÜSS, Sebastian, 1180 Wien (AT)
(74) Vertreter: Gramm, Lins & Partner Patent- und Rechtsanwälte PartGmbB
(86) Internationale Anmeldenummer: PCT/EP2021/060079
(87) Internationale Veröffentlichungsnummer: WO 2021/213976

(56) Entgegenhaltungen:
- DE-A1- 102009 052 891
- DE-A1- 102017 131 190
- DE-B3- 102016 114 075
- DE-B3- 102017 119 490

## Beschreibung

Die Erfindung betrifft ein Verfahren zum Übertragen von Daten, insbesondere zum Konfigurieren eines Prothesensystems, zwischen einem Prothesensystem und einem Eingabegerät, wobei das Prothesensystem mehrere Prothesenkomponenten aufweist, die zumindest eine elektrische und/oder mechatronische Komponente aufweisen, wobei zumindest eine Prothesenkomponenten über eine Schnittstelle mit einem Eingabegerät verbunden wird, sowie ein System zur Durchführung des Verfahrens.

Prothesen ersetzen nicht oder nicht mehr vorhandene Gliedmaßen. Die einfachsten Prothesenkomponenten haben eine rein kosmetische Funktion oder vervollständigen eine Gliedmaße, beispielsweise indem ein distales Fingerglied ersetzt wird. Im Laufe der Zeit wurden die Prothesen komplexer, mehrere Prothesenkomponenten wurden aneinander angeordnet und befestigt und beispielsweise über Gelenke miteinander verbunden. Komplexe mechanische Antriebsvorrichtungen wurden entwickelt, um beispielsweise Prothesenhände oder Prothesenfüße zu bewegen. Hydraulische Dämpfereinrichtungen wurden an Gelenken angeordnet, um das Verhalten von Prothesenkomponenten und Prothesensystemen zu modifizieren, um einen möglichst natürlichen Bewegungsablauf zu ermöglichen. Zur Unterstützung von Bewegungen wurden Antriebe in Prothesenkomponenten integriert, sodass aktive Prothesen entstanden sind. Weiterhin wurden Sensoren an Prothesenkomponenten oder einem Prothesennutzer angeordnet, um das gegenwärtige Bewegungsverhalten zu erfassen und das zukünftige Bewegungsverhalten abzuschätzen und um Einstellungen an Dämpfern und/oder Antrieben zu verändern. Dadurch sind hochkomplexe Prothesensysteme mit mehreren, aneinander angeordneten Prothesenkomponenten entstanden, die eine Vielzahl von mechanischen, elektrischen und mechatronischen Komponenten aufweisen.

Ein Prothesensystem der unteren Extremität kann einen Oberschenkelschaft aufweisen, an dem ein Prothesenkniegelenk, ein Prothesen-Unterschenkel und ein Prothesenfuß befestigt sind. Ein solches Prothesensystem weist beispielsweise zwei oder mehr Gelenke auf, die jeweils mit Dämpfern und/oder Antrieben versehen sein können. Solche Prothesensysteme müssen auf den jeweiligen Patienten individuell angepasst werden, was durch einen Orthopädietechniker erfolgt. Jede einzelne Prothesenkomponente mit den mechatronischen Komponenten muss für den jeweiligen Nutzer ausgewählt und optimiert angepasst werden. Hierbei sind für jede Prothesenkomponente eine Vielzahl von Daten und Kenngrößen einzugeben, beispielsweise nutzerspezifische Daten wie das Gewicht, den Mobilitätsgrad, die Körpergröße und vieles mehr.

Die DE 102017 119 490 B3 betrifft ein Prothesensystem mit mehreren Sensoren und zumindest einer Steuerungseinrichtung, die mit den Sensoren gekoppelt ist und die Sensorsignale verarbeitet. Ein Aktuator in Gestalt eines Motors ist mit der Steuerungseinrichtung gekoppelt und wird auf der Grundlage von Steuerungssignalen aktiviert oder deaktiviert. Dadurch wird zumindest eine Prothesenkomponente verlagert oder deren Verlagerung wird angehalten. Um die Funktionsfähigkeit des Prothesensystems zu überprüfen, ist in der Steuerungseinrichtung ein Standardprogramm hinterlegt oder abrufbar, das jedem Sensor eine Aktuatoraktion unabhängig von der Dauer und/oder Intensität des Sensorsignals zuordnet.

Aufgabe der vorliegenden Erfindung ist es, ein Verfahren und ein System zum Übertragen von Daten, insbesondere zum Konfigurieren eines Prothesensystems bereitzustellen, durch die eine verbesserte und schnellere Anpassung des Systems oder unterschiedlicher Systemkomponenten an den jeweiligen Nutzer erreicht werden kann.

Erfindungsgemäß wird diese Aufgabe durch ein Verfahren mit den Merkmalen des Hauptanspruches und ein System mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Verfahren zum Übertragen von Daten zwischen einem Prothesensystem und einem Eingabegerät, insbesondere zum Konfigurieren eines Prothesensystems, wobei das Prothesensystem mehrere Prothesenkomponenten aufweist, die zumindest eine elektronische und/oder mechatronische Komponente aufweisen, wobei zumindest eine Prothesenkomponente über zumindest eine Schnittstelle mit einem Eingabegerät verbunden wird, sieht vor, dass zumindest zwei elektronischen und/oder mechatronischen Komponenten gemeinsam in einem mit den Prothesenkomponenten verbundenen Zustand mit dem Eingabegerät verbunden und über das eine Eingabegerät Daten übermittelt oder ausgetauscht werden, wobei mehrere elektronische Komponenten gleichzeitig mit Daten versorgt werden und/oder mehrere elektronische Komponenten gleichzeitig Daten übermitteln. Gegenwärtig ist es notwendig, jede Prothesenkomponente einzeln zu konfigurieren, wozu jede einzelne Prothesenkomponente mit einer mechatronischen Komponente mit dem Eingabegerät verbunden wird, damit die notwendigen Daten übermittelt oder ausgetauscht werden. Über eine Eingabemaske wird für jede Prothesenkomponente und gegebenenfalls für jede mechatronische Komponente eine Vielzahl von Abfragen durchgeführt, um eine an den Patienten angepasste Konfiguration durch den Datenaustausch oder die Datenübertragung zu erreichen. Insbesondere bei beidseitig versorgten Patienten kann dies einen extremen Zeitaufwand bedeuten. Erfindungsgemäß ist vorgesehen, dass alle elektronischen und/oder mechatronischen Komponenten gemeinsam mit den Prothesenkomponenten mit dem Eingabegerät verbunden und über das Eingabegerät eine Datenübermittlung oder ein Datenaustausch durchgeführt werden, sodass sich zum Beispiel ein erhöhter Einstellkomfort und eine erhöhte Bedienungsfreundlichkeit für den Orthopädietechniker und den Patienten ergeben. Die Zeit, die zur Datenübermittlung oder zum Datenaustausch, z.B. bei der Konfiguration eines Prothesensystems benötigt wird, kann sich halbieren oder auf bis zu 25 % verringern. Darüber hinaus sind für eine Vielzahl von Abfragen automatisierte Eingaben oder Einstellungen möglich, sodass redundante Eingaben für die jeweiligen Komponenten nicht mehr anfallen. Das Eingabegerät kann dabei auch eine mit einer Prothesenkomponente gekoppelte Datenverarbeitungseinrichtung sein, die zum Beispiel mit Sensoren verbunden ist und auf deren Grundlage Daten an andere elektronische und/oder mechatronische Komponenten übermittelt. Die Datenübermittlung oder der Datenaustausch, z.B. bei der Konfiguration kann somit über eine externe Datenverarbeitungseinrichtung wie Computer, Tablet, Mobiltelefon oder dergleichen erfolgen oder über eine in einer Prothesenkomponente integrierte Datenverarbeitungseinrichtung. Auch ein Prothesengelenk kann ein Eingabegerät sein, beispielsweise wenn es über seine Sensorik Informationen enthält, die relevant für das Bewegungs- oder Gangverhalten sind und entsprechend die anderen Prothesenkomponenten ansteuert. Die Informationen können beispielsweise das Gewicht des Nutzers, die Gehgeschwindigkeit, die Schrittlänge und dergleichen sein, die über Drucksensoren, Winkelsensoren, Lagesensoren, Momentensensoren und/oder IMU erhalten werden können. Unter Konfiguration wird insbesondere nicht nur die Zusammenstellung von Bauteilen und Softwarekomponenten, sondern auch das Einrichten der Soft- und Hardware und die Abstimmung der Komponenten untereinander und Anpassung aneinander verstanden, wobei jeder Aspekt auch einzeln als Konfiguration anzusehen ist. Die Konfiguration aller Komponenten kann über ein einziges Eingabegerät erfolgen, wobei eine direkte Datenübermittlung oder ein direkter Datenaustausch, z.B. bei der Konfiguration durch eine Verbindung der Komponenten mit dem Eingabegerät über die Schnittstellen oder eine indirekte Datenübermittlung oder ein indirekter Datenaustausch, z.B. bei der Konfiguration über eine Schnittstelle zu einer Komponente, von der aus die weitere Datenübermittlung oder der Datenaustausch oder die weitere Konfiguration erfolgt, stattfinden kann.

Eine Weiterbildung der Erfindung sieht vor, dass zumindest zwei Prothesenkomponenten, insbesondere alle Prothesenkomponenten des Prothesensystems, aneinander festgelegt und anschließend mit Daten versehen und konfiguriert werden oder ein Datenaustausch stattfindet. Dadurch ist es möglich, Wechselwirkungen zwischen den einzelnen Prothesenkomponenten und deren mechatronischen Komponenten automatisch zu erkennen und bei der Datenübermittlung oder dem Datenaustausch oder der Konfiguration zu berücksichtigen. Werden die beiden Prothesenkomponenten oder die jeweiligen Prothesenkomponenten aneinander festgelegt, kann dies durch Sensoren, Sender, Transponder oder Schalter automatisch in dem Eingabegerät erfasst werden. An den mechanischen Kopplungseinrichtungen, beispielsweise Pyramidenadapter oder dergleichen, können Kontakte ausgebildet sein, die das Vorhandensein der angeschlossenen Prothesenkomponente detektieren. Ebenso kann eine Information übermittelt werden, welche Prothesenkomponente an der jeweiligen anderen Prothesenkomponente angeordnet ist. Für jede Prothesenkomponente können unterschiedliche Abfragen oder Eingaben hinterlegt sein, die bei einer Kombination der beiden Prothesenkomponenten nur einmal eingegeben werden müssen. Nachdem detektiert worden ist, dass die beiden Prothesenkomponenten oder mehrere Prothesenkomponenten aneinander festgelegt worden sind, müssen eine Vielzahl von Daten nur ein einziges Mal eingegeben werden, um das gesamte Prothesensystem zu konfigurieren. Darüber hinaus ist es möglich, dass bestimmte Eingabefelder eingeblendet oder ausgeblendet werden, die nur in der jeweiligen Kombination zweier oder mehrerer Prothesenkomponenten notwendig sind bzw. nicht benötigt werden. Jede Prothesenkomponente kann mehrere elektronische und/oder mechatronische Komponenten aufweisen, die ebenfalls automatisch identifiziert werden können.

Die Prothesenkomponenten können über zumindest eine drahtlose Schnittstelle mit dem Eingabegerät verbunden werden, wodurch es für den Orthopädietechniker und den Nutzer der Prothesenkomponenten einfacher wird, die Datenübermittlung oder den Datenaustausch oder die Konfiguration durchzuführen. Grundsätzlich ist es auch möglich, dass die Datenübermittlung oder der Datenaustausch oder die Konfiguration entfernt von dem Patienten durchgeführt wird, beispielsweise nach Wartungsarbeiten oder zur Anpassung, wenn aufgrund von Veränderungen in dem Bewegungsverhalten oder den Möglichkeiten des Patienten eine andere Prothesenkomponente sinnvoll erscheint. Dem jeweiligen Prothesennutzer und/oder Orthopädietechniker wird dann die neue Prothesenkomponente zugeschickt. Nach der Montage werden dann die elektrischen und/oder mechatronischen Komponenten auf die vorgenommene Einstellung hin optimiert und konfiguriert und an die bereits vorhandenen Prothesenkomponenten angepasst. Die Anpassung und/oder Einstellung kann durch den Orthopädietechniker und/oder den Prothesennutzer erfolgen.

Über das Eingabegerät können Konfigurationsparameter des Prothesensystems und/oder Befehle, insbesondere Kommandos zur Ausführung von Bewegungen übermittelt oder ausgetauscht werden.

Über das Eingabegerät können vorab ermittelte oder gespeicherte nutzerbezogene Daten, insbesondere zur Konfiguration an die jeweilige Prothesenkomponente oder die Prothesenkomponenten übermittelt werden. Sind nutzerbezogene Daten bereits bei dem Orthopädietechniker vorhanden oder in einer Datenbank abrufbar, beispielsweise bei dem Hersteller der Prothesenkomponenten, dem Arzt, dem Physiotherapeuten oder einer anderen Institution, die den Nutzer des Prothesensystems betreut, können die in dem Eingabegerät gespeichert oder diesem übermittelt werden, sodass eine Verringerung der Eingabedauer erreicht werden kann.

Die Schnittstelle zu dem Eingabegerät kann bidirektional ausgebildet sein, sodass Informationen nicht nur von dem Eingabegerät an die Prothesenkomponente oder Prothesenkomponenten übermittelt werden, sondern umgekehrt, dass auch Informationen über die Prothesenkomponenten und die darin befindlichen elektronischen und/oder mechatronischen Komponenten von diesen an das jeweilige Eingabegerät übermittelt werden. Diese Informationen können mit den in dem Eingabegerät abgelegten Abfragemasken abgeglichen oder anderweitig ausgewertet und/oder verarbeitet werden. Ebenfalls ist es möglich, dass nutzerbezogene Daten, die bereits vorliegen, automatisch in Abhängigkeit von der jeweils ermittelten und identifizierten Prothesenkomponente verarbeitet werden, sodass eine beschleunigte Datenübermittlung oder ein beschleunigter Datenaustausch oder eine beschleunigte Konfiguration möglich wird. Über die bidirektionale Schnittstelle ist es zudem möglich, dass der Orthopädietechniker unmittelbar eine Rückmeldung über das Verhalten des Prothesensystems als Reaktion auf die vorgenommenen Einstellungen erhält.

Über das Eingabegerät können die mechatronischen Komponenten individuell oder in Abhängigkeit von der festgestellten bzw. ausgewählten Kombination der Prothesenkomponenten gemeinsam konfiguriert werden, wobei die Datenübermittlung oder der Datenaustausch oder die Konfiguration über ein Eingabegerät erfolgt. Dadurch wird dem Orthopädietechniker oder derjenigen Person, die die Datenübermittlung oder den Datenaustausch oder die Konfiguration vornimmt, die Möglichkeit gegeben, eine Anpassung an die Wünsche und Fähigkeiten des jeweiligen Prothesennutzers vorzunehmen.

Über das Eingabegerät können verschiedene Parameter der Prothesenkomponenten und der mechatronischen Komponenten verändert werden, wobei die Parameter oder Parameterbereiche, die verändert werden können, in Abhängigkeit von der vorhandenen Prothesenkomponentenkombination auf dem Eingabegerät angezeigt werden. Dies erleichtert die Datenübermittlung oder den Datenaustausch oder die Konfiguration, da nur diejenigen Parameter oder Parameterbereiche angezeigt werden, die tatsächlich sinnvoll und möglich sind. So können beispielsweise unterschiedliche Dämpfungsniveaus in der Standphasendämpfung eines Prothesenkniegelenkes bei unterschiedlichen Prothesenfüßen sinnvoll sein, sodass einige Dämpfungsbereiche von vornherein ausgeschlossen werden können oder müssen. Dadurch wird die Gefahr einer Fehleinstellung oder Fehlkonfiguration vermieden oder verringert.

Die Datenübermittlung oder der Datenaustausch, insbesondere zur Konfiguration der elektrischen und/oder mechatronischen Komponente bzw. der Komponenten, kann unmittelbar über das Eingabegerät erfolgen, das über zumindest eine Schnittstelle mit der jeweiligen Prothesenkomponente und damit mit der jeweiligen elektronischen und/oder mechatronischen Komponente verbunden ist. Wenn nur eine Schnittstelle vorhanden ist oder die Nutzung nur einer Schnittstelle für vorteilhaft erachtet wird, beispielsweise um Bauraum einzusparen, Anschlüsse nicht vorsehen zu müssen oder eine drahtlose Verbindung mit einer Komponente gewünscht wird, kann die die Datenübermittlung oder der Datenaustausch oder Konfiguration auch unter Zwischenschaltung einer oder mehrerer Prothesenkomponenten bzw. elektronischer und/oder mechatronische Komponenten erfolgen. Die Komponenten untereinander sind verbunden, gegebenenfalls sowohl mechanisch als auch informationstechnisch. Bei beidseitig versorgten Patienten findet die Kopplung aller Komponenten vorteilhafterweise über eine drahtlose Verbindung statt. Die indirekte Datenübermittlung oder Konfiguration sieht dann vor, dass das Eingabegerät der einen Schnittstelle an einer Prothesenkomponente verbunden wird und dann über diese Prothesenkomponente Informationen oder Daten, Befehle, Kommandos oder Konfigurationsparameter auf die gekoppelten und/oder verbundenen Prothesenkomponenten verteilt werden

Das System zu Durchführung des oben beschriebenen Verfahrens mit mehreren Prothesenkomponenten, die elektronische und/oder mechatronische Komponenten aufweisen, sieht vor, dass die Prothesenkomponenten gleichzeitig mit einem einzigen Eingabegerät über zumindest eine Schnittstelle verbunden sind und eingerichtet sind, dass mehrere elektronische Komponenten gleichzeitig mit Daten versorgt werden und/oder mehrere elektronische Komponenten gleichzeitig Daten übermitteln. Die mehreren Prothesenkomponenten sind aneinander anordenbar und befestigbar, wobei zumindest zwei Prothesenkomponenten aneinander festgelegt sein können, wenn eine Datenübermittlung, ein Datenaustausch oder eine Konfiguration stattfindet. Die Schnittstelle kann als drahtlose Schnittstelle, insbesondere als eine bidirektionale Schnittstelle ausgebildet sein. Grundsätzlich ist es auch möglich, dass eine Kabelverbindung zu einer oder mehrerer der Prothesenkomponenten vorhanden ist, wenn eine Datenübermittlung, ein Datenaustausch oder eine Konfiguration stattfindet.

In dem Eingabegerät können nutzerbezogene Daten abgelegt sein, die der Konfiguration aller Prothesenkomponenten und der den Prothesenkomponenten zugeordneten mechatronischen Komponenten zugrunde gelegt werden. Das Eingabegerät kann als ein Computer, Tablet, Mobiltelefon oder eine in einer Prothesenkomponente integrierte Datenverarbeitungseinrichtung ausgebildet sein.

Nachfolgend wird die Erfindung anhand der Figuren näher erläutert. Es zeigen:
- Figur 1 -: ein Prothesensystem mit mehreren Prothesenkomponenten; sowie
- Figur 2 -: ein System aus Prothesensystem und Eingabegerät.

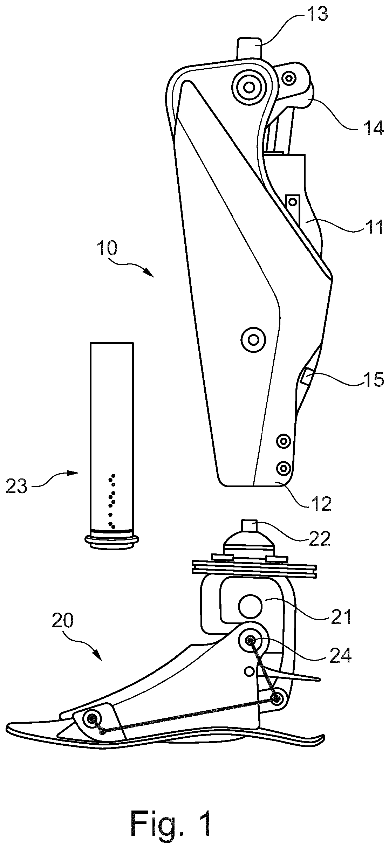

**In** der Figur 1 ist in einer Seitenansicht ein Prothesensystem mit zwei Prothesenkomponenten 10, 20 dargestellt. Eine erste Prothesenkomponente 10 ist als Prothesen-unterschenkel mit einem Prothesenkniegelenk ausgebildet. Das Prothesenkniegelenk weist ein Oberteil auf, das schwenkbar um eine Schwenkachse 14 an einem Unterteil gelagert ist. Innerhalb des Unterteils ist eine erste mechatronische Komponente 11 angeordnet, die als elektronisch gesteuerte, hydraulische Dämpfereinrichtung ausgebildet ist. Innerhalb der mechatronischen Komponente 11 sind Aktuatoren angeordnet, die auf Grundlage von Sensordaten, die z.B. an der ersten Prothesenkomponente 10 angeordnet und mit einer Steuerungseinrichtung, die der mechatronischen Komponente 11 zugeordnet ist, verbunden sind, aktiviert werden. Über die Aktuatoren werden Einstellungen verändert oder Ventile geschlossen oder geöffnet oder Komponenten aktiv verlagert. An dem proximalen Ende der ersten Prothesenkomponente 10 ist ein Pyramidenadapter 13 angeordnet, über den es möglich ist, das Prothesenkniegelenk als Teil der ersten Prothesenkomponente 10 mit einem nicht dargestellten Oberschenkelschaft zu verbinden. An dem distalen Ende der ersten Prothesenkomponente 10 ist eine Befestigungseinrichtung 12 angeordnet oder ausgebildet, die zur Aufnahme eines Pyramidenadapters 22 dient, der in dem proximalen Ende der zweiten Prothesenkomponente 20 in Gestalt eines Prothesenfußes angeordnet ist. In vielen Fällen ist ein Rohradapter 23 oder Unterschenkelrohr zur Kopplung des Prothesenkniegelenkes 10 oder des Unterschenkelteils mit dem Prothesenfuß 20 notwendig.

Der Prothesenfuß 20 weist ein Fußteil, ein Knöchelteil und eine Knöchelgelenkachse 24 auf, um die das Knöchelteil relativ zu einem Fußteil verschwenkt werden kann. Innerhalb des Knöchelteils ist eine weitere mechatronische Komponente 21 angeordnet, die ebenfalls elektronisch gesteuert werden kann. Auf der Basis von Sensordaten werden innerhalb des Prothesenfußes 20 angeordnete Dämpfer oder Antriebe verstellt oder aktiviert bzw. deaktiviert. Die Aufbereitung der Sensordaten kann separat in den jeweiligen Prothesenkomponenten 10, 20 oder gemeinsam in einer gemeinsamen Steuerungseinrichtung erfolgen, indem die Sensordaten aufbereitet und ausgewertet werden. Die Steuerungseinrichtung weist zumindest einen Mikroprozessor auf, der in der Lage ist, während der Benutzung der Prothesenkomponenten 10, 20 die elektronischen Sensordaten zu erfassen, aufzunehmen, zu verarbeiten und in Steuerungssignale für die jeweiligen Antriebe oder Aktuatoren umzuwandeln. Ebenfalls sind Speicherelemente in der Steuerungseinrichtung oder den Steuerungseinrichtungen vorgesehen, um Daten oder Programme zu speichern. Der jeweiligen Prothesenkomponente 10, 20 können separate und/oder gemeinsame Energiespeicher zugeordnet sein.

Die beiden Prothesenkomponenten 10, 20 können mechanisch aneinander festgelegt werden. Dies geschieht durch das Einführen des proximalen Pyramidenadapters 22 des Prothesenfußes 20 in eine entsprechende Befestigungseinrichtung 12 an dem distalen Ende des Unterschenkelteils bzw. der ersten Prothesenkomponente 10 oder eines Rohradapters 23.

In der jeweiligen Prothesenkomponente können Marker oder Identifikatoren angeordnet sein, die es ermöglichen, dass bei einer mechanischen Verbindung zweier Prothesenkomponenten miteinander der jeweiligen Steuerungseinrichtung mitgeteilt wird oder diese erkennt, welche Kombination an Prothesenkomponenten gerade vorhanden ist. Die Marker oder Identifikatoren können auch über ein Eingabegerät drahtlos abgefragt werden, so dass dem Eingabegerät bekannt ist, welche Komponenten insgesamt für welchen Patienten konfiguriert werden sollen. Die Marker können elektronische Marker, z.B. RFID oder Transponder, optische Marker in Gestalt von QR-Codes oder andere Informationsträger sein, die automatisch auslesbar sind.

In dem dargestellten Ausführungsbeispiel ist an der ersten Prothesenkomponente 10 eine Sende- und Empfangseinrichtung 15 angeordnet, mit der es möglich ist, Informationen zu übermitteln, beispielsweise Daten eines Steuerungsprogrammes zu empfangen, Änderungen oder Updates aufzuspielen oder Nutzungsdaten, die während der Benutzung der Prothese aufgenommen worden sind, an externe Geräte zu übermitteln. Die Sende- und Empfangseinrichtung 15 kann als drahtlose Schnittstelle oder als kabelgebundener Anschluss, beispielsweise als Buchse, ausgebildet sein.

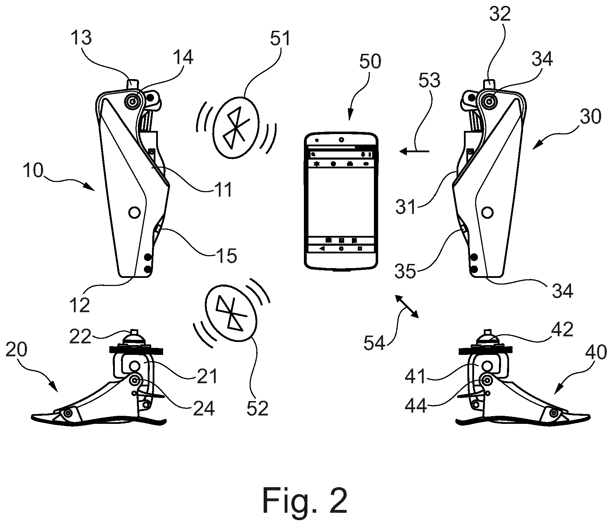

In der Figur 2 ist ein erfindungsgemäßes System mit insgesamt vier Prothesenkomponenten 10, 20, 30, 40 dargestellt, mit denen eine beidseitige Versorgung eines Patienten möglich ist. Dargestellt sind zwei Prothesenfüße 20, 40 und zwei Unterschenkelkomponenten 10, 30 mit Prothesenkniegelenken und proximalen Anschlusseinrichtungen 13, 32. Der grundsätzliche Aufbau der einzelnen Komponenten entspricht dem in der Figur 1 erläuterten Aufbau.

Zur Konfiguration des Gesamtsystems aus insgesamt vier Prothesenkomponenten 10, 20, 30, 40 ist ein einziges Eingabegerät 50 vorgesehen, das in dem dargestellten Ausführungsbeispiel als Tablet dargestellt ist. Alternative Eingabegeräte 50 sind möglich, beispielsweise ein Smartphone, Computer, Laptop, StandPC, eine Smart-Watch oder ein anderes, sogenanntes Smart Device. Das Eingabegerät 50 fungiert als Master, während die vier Prothesenkomponenten 10, 20, 30, 40 als Slaves ausgebildet sind, die über das Eingabegerät 50 mit Daten oder Informationen versorgt werden. Die Verbindung zwischen den Prothesenkomponenten 10, 20, 30, 40 und dem Eingabegerät 50 erfolgt über drahtlose Schnittstellen 51, 52, 53, 54, die symbolisch dargestellt sind. Beispielweise können Daten über eine Funkverbindung in einem lizenzfreien ISM-Band, mit einer sogenannten Bluetooth-Verbindung übertragen werden. Die Verbindung kann bidirektional aufgebaut sein, alternativ können andere drahtlose Schnittstellen oder auch kabelgebundene Schnittstellen angeordnet sein, um über Datenaustauscheinrichtungen oder Sende- und Empfangseinrichtungen 15, 35 Daten zu übermitteln oder auszutauschen. Solche Sende- und Empfangseinrichtungen können auch an den Prothesenfüßen 20, 40 angeordnet oder ausgebildet sein. Damit ist auch eine direkte, bidirektionale Kommunikation oder ein Datenaustausch zwischen den Prothesenkomponenten 10, 20, 30, 40 möglich, ohne dass ein Eingabegerät 50 verwendet werden muss.

Während es bei bisherigen mechatronischen Komponenten bislang notwendig war, zur Datenübermittlung, zum Datenaustausch oder zur Konfiguration jede einzelne Komponente mit dem Eingabegerät 50 zu verbinden und separat einzustellen oder mit Daten zu versorgen, ist erfindungsgemäß vorgesehen, dass alle mechatronischen Komponenten 11, 21, 31, 41 gleichzeitig mit einem Eingabegerät 50, das als Master-Mobilgerät ausgebildet ist, direkt oder indirekt verbunden werden. Somit verfügt der Orthopädietechniker über eine Möglichkeit, sämtliche einzugebenden Daten gleichzeitig an alle Prothesenkomponenten 10, 20, 30, 40 zu übermitteln und dadurch ein schnelles Feedback der Anwender oder Nutzer des Prothesensystems zu ermöglichen. Bei einer bidirektionalen Kommunikation verfügt der Orthopädietechniker umgekehrt über die Möglichkeit, sämtliche Daten von allen Prothesenkomponenten 10, 20, 30, 40 gleichzeitig mit einer Abfrage zu erhalten und dadurch selbst eine schnelle Rückmeldung über das Prothesensystem zu erhalten. Darüber hinaus können durch einen automatischen Abgleich der vorhandenen Komponenten in einer Datenbank, auf die das Eingabegerät zugreift, Parameter und Parameterbereiche vorgegeben sowie nicht mehr notwendige Mehrfacheingaben ausgeblendet werden, sodass nicht für alle Prothesenkomponenten 10, 20, 30, 40 immer alle nutzerspezifischen Daten eingegeben werden müssen. Durch die bidirektionalen Schnittstellen ist es möglich, dass das Eingabegerät 50 die vorhandenen und miteinander kombinierten Prothesenkomponenten 10, 20, 30, 40 erkennt und Parameter und Parameterbereiche, die einstellbar sind, vorschlägt oder vorgibt. Der Orthopädietechniker kann wählen, ob eine Gesamtsystemeinstellung oder eine individuelle Einstellung der einzelnen Prothesenkomponenten 10, 20, 30, 40 erfolgen soll.

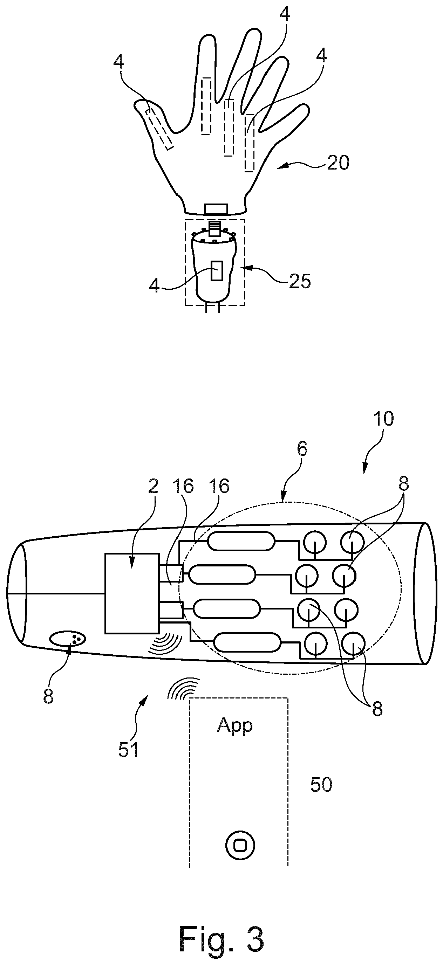

Figur 3 zeigt eine schematische Darstellung einer Ausführungsform des Prothesensystems einer ersten Prothesenkomponente 10 in Gestalt eines Unterarmschaftes oder eine sogenannten Brace, die als ein Modell des eigentlichen Unterarmschaftes dient und noch nicht die vollständige Funktionalität eines Unterarmschaftes aufweist. Eine solche Brace dient zu Trainingszwecken und weist nicht notwendigerweise Befestigungseinrichtungen für die zweite Prothesenkomponente 20 auf, die in Gestalt einer Prothesenhand ausgebildet ist. Die zweite Prothesenkomponente 20 ist in dem dargestellten Ausführungsbeispiel nicht an der Brace oder dem Unterarmschaft als erste Prothesenkomponente 10 mechanisch befestigt, sondern auf einem Träger 25 angeordnet. Der Träger 25 weist Anschlüsse oder eine Schnittstelle für Steuerungssignale auf und kann auch einen Antrieb 4 für eine drehbare Lagerung der Prothesenhand 20 oder gegebenenfalls Antriebe für bewegliche Prothesenfinger aufweisen. In dem dargestellten Ausführungsbeispiel sind innerhalb der zweiten Prothesenkomponente 20 in Gestalt einer Prothesenhand drei Antriebe 4 für die Aktuierung der Prothesenfinger und des Prothesendaumens angeordnet, die über Steuerungssignale aktiviert und deaktiviert werden können. Die erste Prothesenkomponente 10 weist eine erste Sensoranordnung 6 mit einer Mehrzahl von Elektrodenpaaren 8 sowie eine Steuerungseinrichtung 2 in Gestalt einer elektronischen Datenverarbeitungseinrichtung auf. In der Steuerungseinrichtung 2 sind die üblichen elektrischen und elektronischen Komponenten vorhanden, beispielsweise Speichereinrichtungen, Datenverarbeitungseinrichtungen, Prozessoren, Verstärker und Energiespeicher und ggf. eine Eingabeeinrichtung und eine Ausgabeeinrichtung und/oder ein Bildschirm. Separate Energiespeicher können ebenfalls vorgesehen sein. Darüber hinaus ist in der Steuerungseinrichtung 2 eine drahtlose Schnittstelle 51 eingerichtet, über die die erste Prothesenkomponente 10 mit dem Eingabegerät 50 verbunden werden kann um einen Datenaustausch entweder in eine Richtung oder einen bidirektionalen Datenaustausch zu ermöglichen.

Der in dem Träger 25 angeordnete Antrieb 4 dient dazu, die erste Prothesenkomponente 10 oder Prothesenhand relativ zu einem definitiven Unterarmschaft des Prothesensystems um die Längsachse des Unterarmschaftes zu verdrehen.

Die in der Brace angeordnete Sensoranordnung 6 weist vier Elektrodenpaare 8 auf. Diese sind beispielsweise als einzelne Elektrodenpaare 8 ausgebildet, die jeweils auf der Haut eines Prothesenträgers befestigt werden können. Gemäß einer weiteren Ausführungsform sind die Elektrodenpaare 8 auf einem nicht dargestellten Prothesenliner aufgebracht, beispielsweise aufgeklebt oder integral mit einem Liner verbunden oder ausgebildet. Die Elektrodenpaare 8 sind jeweils über Sensorleitungen 16 mit der Steuerungseinrichtung 2 verbunden. Über die Sensorleitungen 16 werden die von den Elektroden 8 erfassten Signale an die Steuerungseinrichtung 2 geleitet.

Neben den dargestellten Elektrodenpaaren 8 als Sensoren zur Erfassung myoelektrischer Signale sind andere Sensoren 8 an dem Unterarmschaft angeordnet, die als Inertialsensoren, IMU, Raumlagesensoren, Beschleunigungssensoren, Kraftsensoren, Winkelsensoren, Temperatursensoren oder andere Sensoren ausgebildet sein können. Es können auch mehrere Sensoren 8 an der orthopädietechnischen Einrichtung angeordnet sein, die unterschiedliche Messgrößen oder Bedingungen erfassen. Ebenso können Sensoren 8 in der Prothesenhand angeordnet sein, beispielsweise Positionssensoren, die die Stellung der Prothesenfinger erfassen. Die Signale der Sensoren 8 werden in der Steuerungseinrichtung E ausgewertet.

Die Steuerungseinrichtung 2 steht vorliegend in drahtloser Verbindung, beispielsweise über Funk, mit einem Eingabegerät 50. Über dieses Eingabegerät 50 können beispielsweise erhaltene Signale der Sensoranordnung 6 visualisiert werden. Die Steuerungseinrichtung 2 kann ebenfalls drahtlos mit der Prothesenhand als zweite Prothesenkomponente sein. Ebenso findet eine Verbindung über Funk beispielsweise mit dem Träger 25 statt. Alternativ kann eine kabelgebundene Verbindung zwischen der Steuerungseinrichtung 2 und der zweiten Prothesenkomponente 20 bestehen.

Es besteht ebenfalls die Möglichkeit, dass eine große räumliche Trennung zwischen der zweiten Prothesenkomponente 20 und der Steuerungseinrichtung 2 bzw. der ersten Prothesenkomponente 10 besteht. Die erste Prothesenkomponente 10 oder Brace kann von dem Nutzer angelegt werden. Die zweite Prothesenkomponente 20 kann beispielsweise über das Internet mit der Steuerungseinrichtung 2 gekoppelt sein, und eine Konfiguration des gesamten Prothesensystems kann über das Eingabegerät 50 erfolgen. Das Eingabegerät 50 ist nur mit der ersten Prothesenkomponente 10 oder der Brace direkt verbunden, die Verbindung mit der zweiten Prothesenkomponente 20 erfolgt über die erste Prothesenkomponente 10, also indirekt.

Durch die räumliche Entkopplung der Prothesenkomponenten 10, 20 voneinander ist es möglich, dass ein Orthopädietechniker in seiner Werkstatt mechanische Anpassungen der Prothesenhand 20 ausführt, während der Nutzer über die erste Prothesenkomponente 10 die individuellen Anpassungen ausführt.

Alternativ ist es vorgesehen, dass das Eingabegerät 50 eine Vermittlungsstellung zwischen den beiden Prothesenkomponenten 10, 20 einnimmt. Durch die bidirektionale Verbindung von Eingabegerät 50 mit der ersten Prothesenkomponente 10 ist es möglich, Sensordaten der Sensoren 8 an das Eingabegerät 50 zu senden. In dem Eingabegerät 50 werden diese Sensordaten verarbeitet und über eine andere Kommunikationsschnittstelle an die entfernte zweite Prothesenkomponente 20 in Gestalt einer sogenannten Tischprothesenhand gesendet. Der Orthopädietechniker oder der Nutzer selbst, z.B. über eine Videoverbindung, kann dann sehen, welche Sensorsignale zu welchen Reaktionen bzw. Steuerungssignalen und damit zu entsprechenden Bewegungen durch die Antriebe 4 an der zweiten Prothesenkomponente 20 führen, ohne dass eine vollständige Ausfertigung eines Prothesenschaftes mit einer festgelegten Positionierung der Sensoren erfolgt sein muss. Das Konfigurieren bzw. die Datenübermittlung oder der Datenaustausch kann somit parallel zu der Anfertigung eines individuellen Unterarmschaftes geschehen, was einen zeitlichen Vorteil bei der Versorgung von Patienten bringt.

## Patentansprüche

1. Verfahren zum Übertragen von Daten zwischen einem Prothesensystem und einem Eingabegerät (50), wobei das Prothesensystem mehrere Prothesenkomponenten (10, 20, 30, 40) aufweist, die zumindest eine elektronische und/oder mechatronische Komponente (2, 4, 8) aufweisen, wobei das Prothesensystem über zumindest eine Schnittstelle (51, 52, 53, 54) mit dem Eingabegerät (50) verbunden wird, wobei zumindest zwei elektronische und/oder mechatronische Komponenten (2, 4, 8) gemeinsam in einem mit den Prothesenkomponenten (10, 20, 30, 40) verbundenen Zustand mit dem Eingabegerät (50) verbunden und über das eine Eingabegerät (50) Daten übermittelt oder ausgetauscht werden, **dadurch gekennzeichnet, dass** mehrere elektronische Komponenten (2, 4, 8) gleichzeitig mit Daten versorgt werden und/oder mehrere elektronische Komponenten (2, 4, 8) gleichzeitig Daten übermitteln.

2. Verfahren nach Anspruch 1, **dadurch gekennzeichnet, dass** zumindest zwei Prothesenkomponenten (10, 20, 30, 40) aneinander festgelegt und anschließend konfiguriert werden.

3. Verfahren nach Anspruch 2, **dadurch gekennzeichnet, dass** die Prothesenkomponenten (10, 20, 30, 40) über zumindest eine drahtlose Schnittstelle (51, 52, 53, 54) mit dem Eingabegerät (50) verbunden werden.

4. Verfahren nach einem der voranstehenden Ansprüche, **dadurch gekennzeichnet, dass** über das Eingabegerät Konfigurationsparameter des Prothesensystems und/oder Befehle, insbesondere Kommandos zur Ausführung von Bewegungen übermittelt oder ausgetauscht werden.

5. Verfahren nach einem der voranstehenden Ansprüche, **dadurch gekennzeichnet, dass** über das Eingabegerät (50) vorab ermittelte oder gespeicherte nutzerbezogene Daten, insbesondere zur Konfiguration, übermittelt werden.

6. Verfahren nach einem der voranstehenden Ansprüche, **dadurch gekennzeichnet, dass** die Schnittstelle (51, 52, 53, 54) bidirektional ausgebildet ist und Informationen über die Prothesenkomponenten (10, 20, 30, 40) an das Eingabegerät (50) übermittelt werden.

7. Verfahren nach einem der voranstehenden Ansprüche, **dadurch gekennzeichnet, dass** über das Eingabegerät (50) die mechatronischen Komponenten (2, 4, 8) individuell oder in Abhängigkeit von der festgestellten Kombination der Prothesenkomponenten (10, 20, 30, 40) gemeinsam konfiguriert werden.

8. Verfahren nach einem der voranstehenden Ansprüche, **dadurch gekennzeichnet, dass** über das Eingabegerät (50) verschiedene Parameter veränderbar sind und Parameter oder Parameterbereiche in Abhängigkeit von der vorhandenen Prothesenkomponentenkombination auf dem Eingabegerät (50) angezeigt werden.

9. Verfahren nach einem der voranstehenden Ansprüche, **dadurch gekennzeichnet, dass** die Übermittlung oder der Austausch von Daten, insbesondere zur Konfiguration der elektrischen und/oder mechatronischen Komponente (2, 4, 8), unmittelbar über das Eingabegerät (50) oder unter Zwischenschaltung einer Prothesenkomponente (10, 20, 30, 40) erfolgt.

10. System zur Durchführung des Verfahrens nach einem der voranstehenden Ansprüche mit mehreren Prothesenkomponenten (10, 20, 30, 40), die elektronische und/oder mechatronische Komponenten (2, 4, 8) aufweisen und gleichzeitig mit einem Eingabegerät (50) über zumindest eine Schnittstelle (51, 52, 53, 54) verbunden sind und eingerichtet sind, gleichzeitig mit Daten versorgt werden und/oder gleichzeitig Daten zu übermitteln.

11. System nach Anspruch 10,
**dadurch gekennzeichnet, dass** zumindest zwei Prothesenkomponenten (10, 20, 30, 40) aneinander festgelegt sind.

12. System nach Anspruch 10 oder 11,
**dadurch gekennzeichnet, dass** die Schnittstelle (51, 52, 53, 54) als drahtlose Schnittstelle, insbesondere als bidirektionale Schnittstelle ausgebildet ist.

13. System nach einem der Ansprüche 10 bis 12,
**dadurch gekennzeichnet, dass** in
dem Eingabegerät (50) nutzerbezogenen Daten abgelegt sind, die der Übermittlung und/oder dem Austausch von Daten, insbesondere zur Konfiguration aller Prothesenkomponenten (10, 20, 30, 40), zugrunde gelegt werden.

14. System nach einem der Ansprüche 10 bis 13,
**dadurch gekennzeichnet, dass** das
Eingabegerät (50) als ein Computer, Tablet, Mobiltelefon oder eine in einer Prothesenkomponente (10, 20, 30, 40) integrierte Datenverarbeitungseinrichtung (2) ausgebildet ist.

## Claims

1. Method for transmitting data between a prosthesis system and an input device (50), wherein the prosthesis system has a plurality of prosthesis components (10, 20, 30, 40) which have at least one electronic and/or mechatronic component (2, 4, 8), wherein the prosthesis system is connected to the input device (50) via at least one interface (51, 52, 53, 54), whreas at least two electronic and/or mechatronic components (2, 4, 8), in a state when connected to the prosthesis components (10, 20, 30, 40), are together connected to the input device (50), and data are transmitted or exchanged via the one input device (50), **characterized in that** multiple electronic components (2, 4, 8) are supplied with data simultaneously and/or multiple electronic components (2, 4, 8) transmit data simultaneously.

2. Method according to Claim 1, **characterized in that** at least two prosthesis components (10, 20, 30, 40) are fastened to each other and then configured.

3. Method according to Claim 2, **characterized in that** the prosthesis components (10, 20, 30, 40) are connected to the input device (50) via at least one wireless interface (51, 52, 53, 54).

4. Method according to one of the preceding claims, **characterized in that** configuration parameters of the prosthesis system and/or instructions, in particular commands for executing movements, are transmitted or exchanged via the input device.

5. Method according to one of the preceding claims, **characterized in that** previously determined or stored user-related data, in particular for the configuration, are transmitted via the input device (50).

6. Method according to one of the preceding claims, **characterized in that** the interface (51, 52, 53, 54) is bidirectional, and items of information are transmitted via the prosthesis components (10, 20, 30, 40) to the input device (50).

7. Method according to one of the preceding claims, **characterized in that** the mechatronic components (2, 4, 8) are configured individually or are configured jointly, according to the detected combination of the prosthesis components (10, 20, 30, 40), via the input device (50).

8. Method according to one of the preceding claims, **characterized in that** various parameters are modifiable via the input device (50), and parameters or parameter ranges are displayed on the input device (50) according to the existing combination of prosthesis components.

9. Method according to one of the preceding claims, **characterized in that** the transmission or the exchange of data, in particular for the configuration of the electric and/or mechatronic components (2, 4, 8), is effected directly via the input device (50) or with interposition of a prosthesis component (10, 20, 30, 40).

10. System for carrying out the method according to one of the preceding claims, having a plurality of prosthesis components (10, 20, 30, 40) which have electronic and/or mechatronic components (2, 4, 8) and are simultaneously connected to one input device (50) via at least one interface (51, 52, 53, 54) and are configured to be supplied with data simultaneously and/or to transmit data simultaneously.

11. System according to Claim 10, **characterized in that** at least two prosthesis components (10, 20, 30, 40) are fastened to each other.

12. System according to Claim 10 or 11, **characterized in that** the interface (51, 52, 53, 54) is designed as a wireless interface, in particular as a bidirectional interface.

13. System according to one of Claims 10 to 12, **characterized in that** user-related data are stored in the input device (50) and form a basis for the transmission and/or the exchange of data, in particular for the configuration of all of the prosthesis components (10, 20, 30, 40).

14. System according to one of Claims 10 to 13, **characterized in that** the input device (50) is designed as a computer, tablet or mobile phone or as a data processing device (2) integrated in a prosthesis component (10, 20, 30, 40).

## Revendications

1. Procédé de transmission de données entre un système prothétique et un appareil d'entrée (50), le système prothétique comprenant plusieurs composants de prothèse (10, 20, 30, 40) qui comprennent au moins un composant électronique et/ou mécatronique (2, 4, 8), le système prothétique étant connecté à l'appareil d'entrée (50) par l'intermédiaire d'au moins une interface (51, 52, 53, 54),
dans lequel au moins deux composants électroniques et/ou mécatroniques (2, 4, 8), dans un état connecté aux composants de prothèse (10, 20, 30, 40), sont connectés ensemble à l'appareil d'entrée (50), et les données sont transmises ou échangées par l'intermédiaire dudit appareil d'entrée (50),
**caractérisé en ce que** plusieurs composants électroniques (2, 4, 8) sont alimentés en données simultanément et/ou plusieurs composants électroniques (2, 4, 8) transmettent les données simultanément.

2. Procédé selon la revendication 1,
**caractérisé en ce qu'**au moins deux composants de prothèse (10, 20, 30, 40) sont fixés l'un à l'autre et sont ensuite configurés.

3. Procédé selon la revendication 2,
**caractérisé en ce que** les composants de prothèse (10, 20, 30, 40) sont connectés à l'appareil d'entrée (50) par l'intermédiaire d'au moins une interface sans fil (51, 52, 53, 54).

4. Procédé selon l'une des revendications précédentes,
**caractérisé en ce que** des paramètres de configuration du système prothétique et/ou des instructions, en particulier des commandes d'exécution de mouvements, sont transmis ou échangés par l'intermédiaire de l'appareil d'entrée.

5. Procédé selon l'une des revendications précédentes,
**caractérisé en ce que** des données relatives à l'utilisateur, déterminées ou enregistrées au préalable, en particulier pour la configuration, sont transmises par l'intermédiaire de l'appareil d'entrée (50).

6. Procédé selon l'une des revendications précédentes,
**caractérisé en ce que** l'interface (51, 52, 53, 54) est conçue de façon bidirectionnelle, et des informations sur les composants de prothèse (10, 20, 30, 40) sont transmises à l'appareil d'entrée (50).

7. Procédé selon l'une des revendications précédentes,
**caractérisé en ce que** les composants mécatroniques (2, 4, 8) sont configurés individuellement ou en commun, en fonction de la combinaison identifiée des composants de prothèse (10, 20, 30, 40), par l'intermédiaire de l'appareil d'entrée (50).

8. Procédé selon l'une des revendications précédentes,
**caractérisé en ce que** différents paramètres peuvent être modifiés par l'intermédiaire de l'appareil d'entrée (50), et les paramètres ou des plages de paramètres sont affichés sur l'appareil d'entrée (50) en fonction de la combinaison existante des composants de prothèse.

9. Procédé selon l'une des revendications précédentes,
**caractérisé en ce que** la transmission ou l'échange de données, en particulier pour la configuration des composants électriques et/ou mécatroniques (2, 4, 8), s'effectue directement par l'intermédiaire de l'appareil d'entrée (50) ou avec interconnexion d'un composant de prothèse (10, 20, 30, 40).

10. Système de mise en œuvre du procédé selon l'une des revendications précédentes, comprenant plusieurs composants de prothèse (10, 20, 30, 40) qui comprennent des composants électroniques et/ou mécatroniques (2, 4, 8) et qui sont connectés simultanément à un appareil d'entrée (50) par l'intermédiaire d'au moins une interface (51, 52, 53, 54) et qui sont conçus pour être alimentés en données simultanément et/ou pour transmettre des données simultanément.

11. Système selon la revendication 10,
**caractérisé en ce qu'**au moins deux composants de prothèse (10, 20, 30, 40) sont fixés l'un à l'autre.

12. Système selon la revendication 10 ou 11,
**caractérisé en ce que** l'interface (51 , 52, 53, 54) est conçue comme une interface sans fil, en particulier comme une interface bidirectionnelle.

13. Système selon l'une des revendications 10 à 12,
**caractérisé en ce que** des données relatives à l'utilisateur sont stockées dans l'appareil d'entrée (50) et servent de base à la transmission et/ou à l'échange des données, en particulier pour la configuration de tous les composants de prothèse (10, 20, 30, 40).

14. Système selon l'une des revendications 10 à 13,
**caractérisé en ce que** l'appareil d'entrée (50) est conçu comme un ordinateur, une tablette, un téléphone mobile ou comme un dispositif de traitement de données (2) intégré dans un composant de prothèse (10, 20, 30, 40).
